(19) Europäisches Patentamt
European Patent Office
Office européen des brevets

(11) Publication number: 0 347 892
A1

(12) **EUROPEAN PATENT APPLICATION**

(21) Application number: 89111327.6

(22) Date of filing: 21.06.89

(51) Int. Cl.4: C07D 309/40 , //A61K7/00

(30) Priority: 21.06.88 JP 153884/88

(43) Date of publication of application:
27.12.89 Bulletin 89/52

(84) Designated Contracting States:
DE FR GB IT

(71) Applicant: Sansho Seiyaku Co., Ltd.
26-7, Oike 2-chome
Ohnojo-shi Fukuoka-ken(JP)

(72) Inventor: Higa, Yoshitaka
754-2, Kokubu
Dazaifu-shi Fukuoka-ken(JP)
Inventor: Nakajima, Kazuo
534, Noda
Noda-shi Chiba-ken(JP)

(74) Representative: Patentanwälte Deufel- Schön-
Hertel- Lewald- Otto
Isartorplatz 6
D-8000 München 2(DE)

(54) **Kojic acid derivative.**

(57) Kojic acid mono-γ-linolenate[2-(6′,9′,12′-octadecatrienoyl)oxymethyl-5-hydroxy-4H-pyran-4-one] is a novel compound which exhibits a meritorious inhibitory effect against the tyrosinase activity and can be used as an active ingredient in whitening cosmetics and in external preparations for chromatosis.

EP 0 347 892 A1

# KOJIC ACID DERIVATIVE

## BACKGROUND OF THE INVENTION

### Field of the Invention

This invention relates to kojic acid mono-γ-linolenate which is a novel compound capable of suppressing the activity of tyrosinase.

### Description of the Prior Art

Kojic acid and isokojic acid are known as compounds capable of inhibiting the formation of melanin. Also known are whitening cosmetics containing these acids as active ingredients (refer to Japanese Patent Publication No. 10447/1986 and No. 59084/1987, for example).

It is also known that mono- and di-esters of kojic acid with some fatty acids show inhibitory action against the formation of melanin (refer to Japanese Patent Publication No. 7961/1985 and No. 60801/1986, for example).

## SUMMARY OF THE INVENTION

The object of this invention is to provide a novel compound, kojic acid mono-γ-linolenate, which shows the meritorious effect to suppress the tyrosinase activity.

This invention is a kojic acid derivative having the following forumula (1):

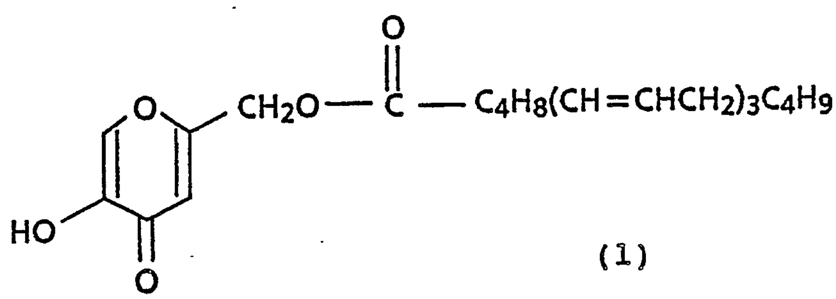

(1)

## BRIEF DESCRIPTION OF THE DRAWINGS

Fig. 1 is the UV absorption spectrum of the compound of this invention;

Fig. 2 is the IR absorption spectrum of the compound of this invention; and

Fig. 3 shows the result of the test on the tyrosinase activity suppressing action of the compound of this invention.

## DETAILED DESCRIPTION OF THE INVENTION

The novel compound (1) of this invention may be prepared by reaction of kojic acid with γ-linolenic acid and has physicochemical properties as shown below.

UV absorption spectrum (solvent: ethanol; λmax 272): As shown in Fig. 1.

IR absorption spectrum: As shown in Fig. 2.

Described below is an example of preparing the compound of this invention.

(Preparative Example)

To a suspension of 0.50 g kojic acid in 10 ml tetrahydrofuran was added 0.74 g aluminum chloride, and the mixture was cooled to a temperature of 5 to 10 °C. A dichloromethane solution (5 ml) containing 1.0 g linolenyl chloride was then added dropwise while the temperature was kept at 5 to 10 °C, and stirred at that temperature for eight hours. The reaction mixture was left as it was overnight and then added to chipped ice containing 2 ml concentrated hydrochloric acid at the temperature below 5 °C. After addition of 15 ml dichloromethane, the resulting mixture was stirred at the temperature below 5 °C for 1.5 hours, and the dichrolmethane layer was collected and washed with saturated aqueous solution of sodium chloride. After washing, chroloform layer was dried by anhydrous sodium sulfate and concentrated. The residue thus obtained was subjected to thin-layer chromatography on silica gel Art-5745 of Merck & Co., Inc.) using a 9:1 mixture of chloroform and ethanol as eluent, and the spots which developed red color by the application of ferric chloride were scraped out and treated with chloroform, giving pure kojic acid mono-γ-linolenate[2-(6′,9′,12′-octadecatrienoyl)oxymethyl-5-hydroxy-4H-pyran-4-one].

The compound of this invention which is capable of suppressing the tyrosinase activity can be used as active ingredient in whitening cosmetics such as cream, emulsions, packs and lotions, and also in external preparations for chromatosis in forms of ointments, lotions etc.

Described below are the test method on the inhibitory action of the compound of this invention against the tyrosinase activity and the result obtained.

(Test Method)

In a test tube, were placed 1 ml of 10 mM DOPA solution, an ethanolic solution containing 0.17, 0.33 or 0.5 mM kojic acid mono-γ-linolenate, and 0.1 M phosphate buffer, making up a total amount of 2.9 ml. The mixture was incubated for three minutes in a thermostatic chamber of 37 °C, 0.1 ml a tyrosinase solution (30,000 G culture supernatant of B16 cells) was added with stirring, and the change in absorbance at 475 nm was measured with time.

(Test Result)

The result obtained is shown in Fig. 3.

As apparent from the result shown above, compared with the control (-●-), 27 % and 40 % inhibition against the tyrosinase activity was observed respectively, when 0.17 mM (-O-) and 0.33 mM (-Δ-) kojic acid mono-γ-linolenate was used.

Consequently, the compound of this invention, kojic acid mono-γ-linolenate ,is a novel compound which exhibits a meritorious inhibitory effect against the tyrosinase activity and can be used as an active ingredient in whitening cosmetics and in external preparations for chromatosis.

**Claims**

1. A kojic acid derivative having the following formula:

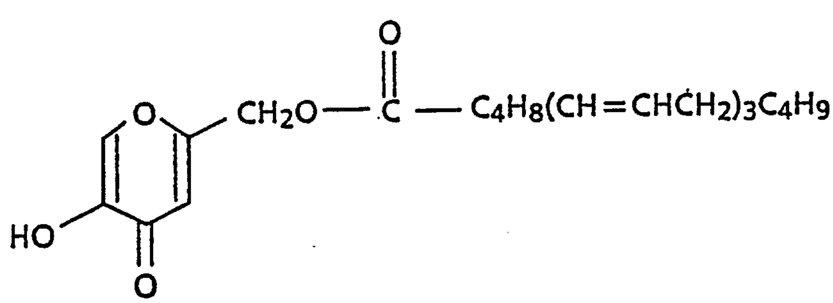

FIG. 1

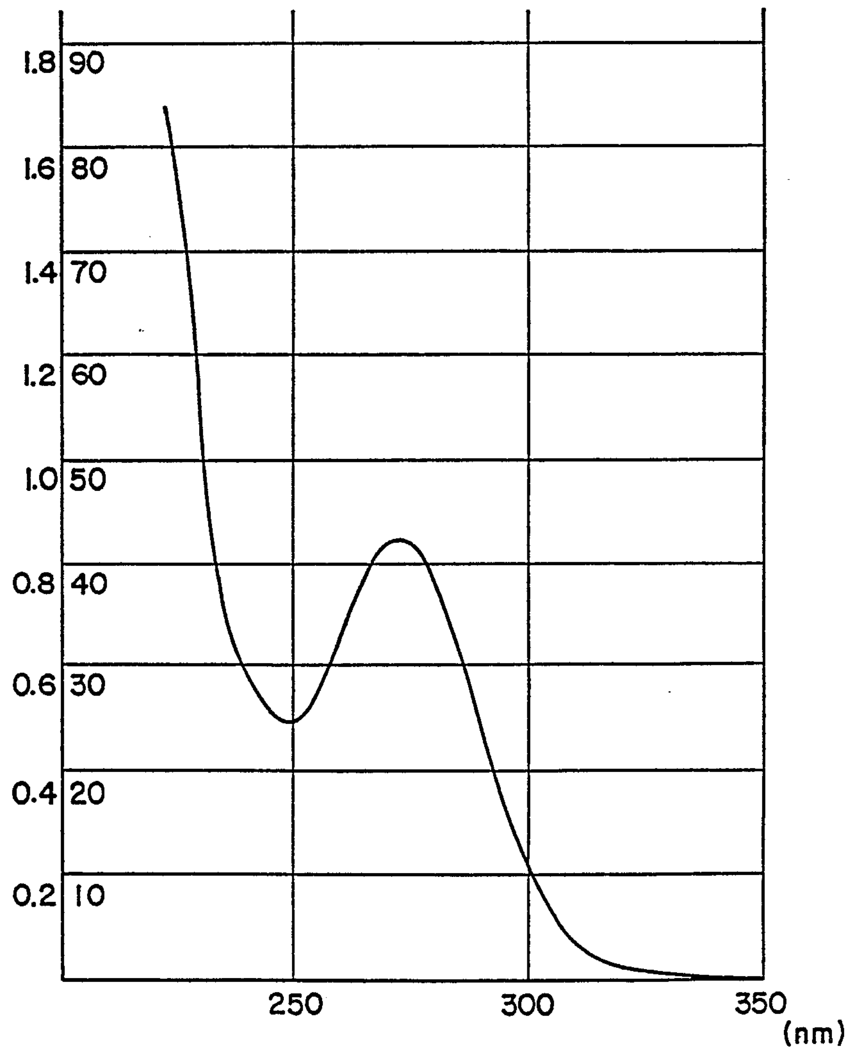

FIG. 2

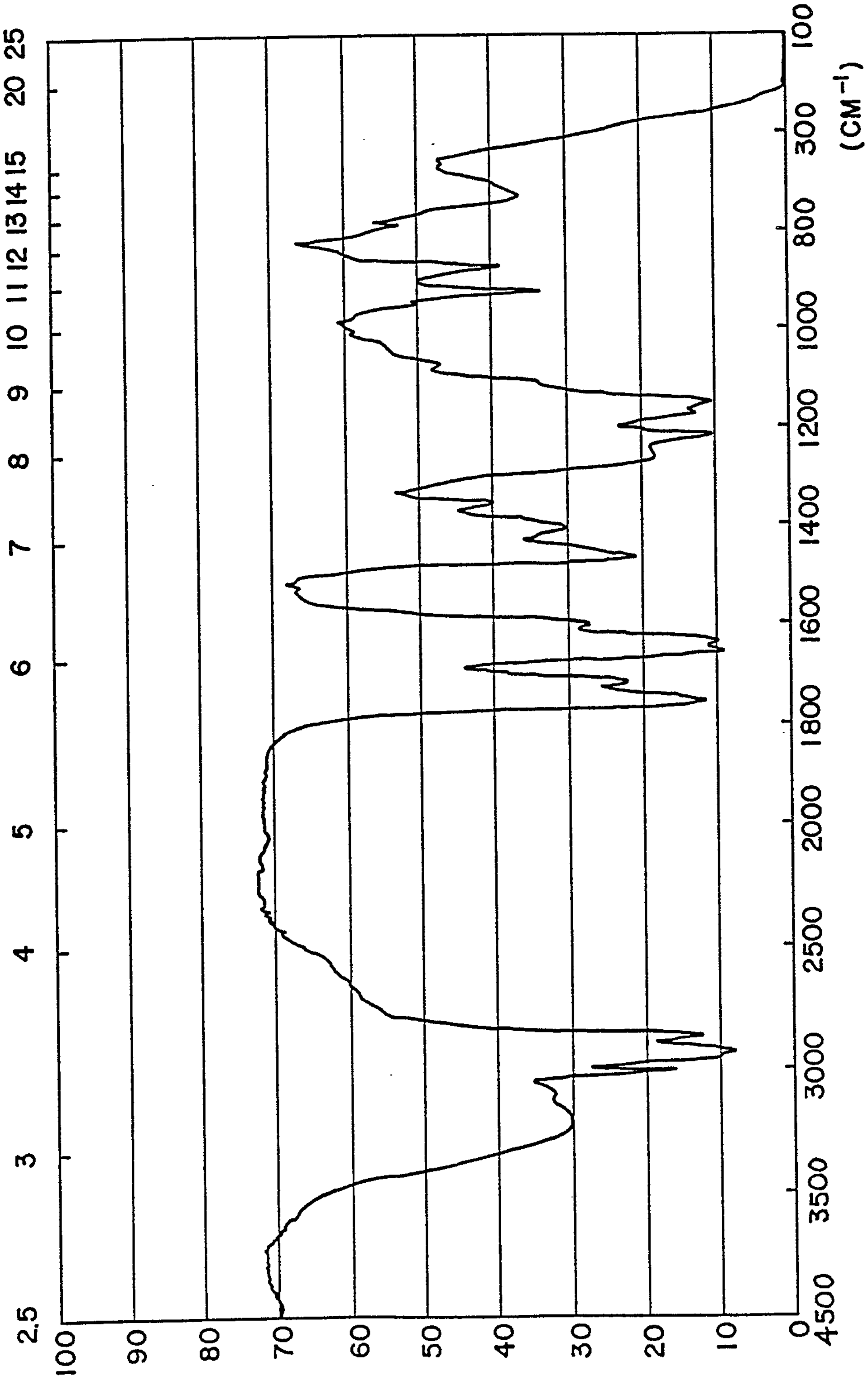
(μm)
2.5
3
4
5
6
7
8
9
10
11
12
13
14
15
20
25
100
90
80
70
60
50
40
30
20
10
0
4500
3500
3000
2500
2000
1800
1600
1400
1200
1000
800
300
100
(CM⁻¹)

FIG. 3

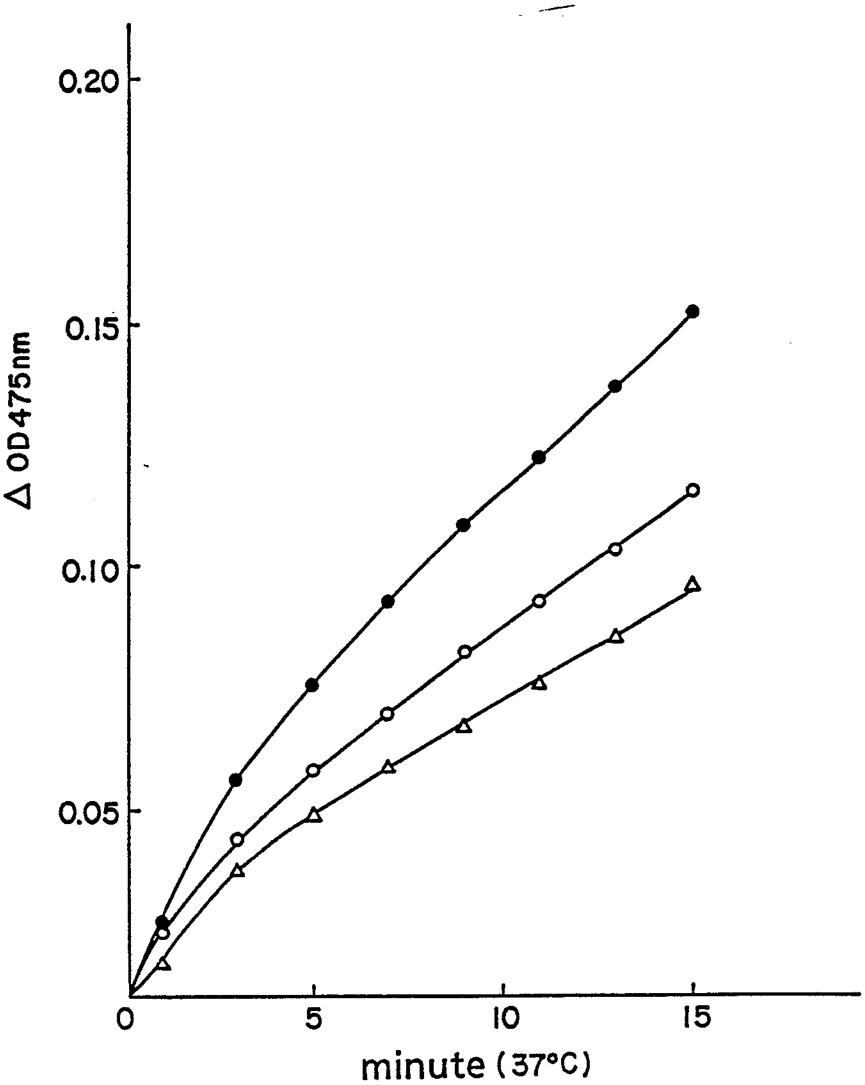

European Patent Office

# EUROPEAN SEARCH REPORT

Application number

EP 89111327.6

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| A | CHEMICAL ABSTRACTS, vol. 108, no. 23, June 6, 1988, Columbus, Ohio, USA MIYANO, M. et al. "(Acyloxy) benzophenones and (acyloxy)-4-pyrones. A new class of inhibitors of human nentrophil elastase" page 640, column 1, Abstract-no. 204 295h & J.Med.Chem. 1988, 31(5), 1052-61 | 1 | C 07 D 309/40 //A 61 K 7/00 |
| A | CHEMICAL ABSTRACTS, vol. 102, no. 7, February 18, 1985, Columbus, Ohio, USA SANSEY PHARMACEUTICAL CO., LTD. "2-(N-Lauroylsarcosyl)-oxymethyl-5-hydroxy-4H-pyran-4-one" page 585, column 1, Abstract-no. 62 080j & Jpn. Kokai Tokkyo Koho JP 59 122 485 (84 122 485) | 1 | TECHNICAL FIELDS SEARCHED (Int. Cl.4) C 07 D 309/00 |
| A | CHEMICAL ABSTRACTS, vol. 100, no. 26, June 25, 1984, Columbus, Ohio, USA SANSEI PHARMACEUTICAL CO., LTD. "Skin-whitening cosmetics" page 329, column 1, Abstract-no. 215 312x & Jpn. Kokai Tokkyo Koho JP 59 33 207 (84 33 207) | 1 | |
| A | CHEMICAL ABSTRACTS, vol. 92, no. 16, April 21, 1980, Columbus, Ohio, USA SANSHO SEIYAKU CO., LTD. | 1 | |

The present search report has been drawn up for all claims

| Place of search | Date of completion of the search | Examiner |
|---|---|---|
| VIENNA | 06-09-1989 | BRUS |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

European Patent Office

# EUROPEAN SEARCH REPORT

Application number

-2-
EP 89111327.6

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
| --- | --- | --- | --- |
| | "White cosmetic materials from the reaction between Kojic acid and aliphatic carboxylic acids" page 404, column 1, Abstract-no. 135 138w & Jpn. Kokai Tokkyo Koho 79 92 632 ---- | | |
| | | | TECHNICAL FIELDS SEARCHED (Int. Cl.4) |

The present search report has been drawn up for all claims

| Place of search | Date of completion of the search | Examiner |
| --- | --- | --- |
| VIENNA | 06-09-1989 | BRUS |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document